(19) Europäisches Patentamt
European Patent Office
Office européen des brevets

(11) Publication number: 0 533 084 A1

# (12) EUROPEAN PATENT APPLICATION

(21) Application number: **92115681.6**

(22) Date of filing: **14.09.92**

(51) Int. Cl.[5]: **A61K 37/64**, A61K 37/02, A61K 37/24

(30) Priority: **16.09.91 US 760552**
**30.04.92 US 876887**

(43) Date of publication of application:
**24.03.93 Bulletin 93/12**

(84) Designated Contracting States:
**PT**

(71) Applicant: **SCHERING CORPORATION**
**2000 Galloping Hill Road**
**Kenilworth New Jersey 07033(US)**

(72) Inventor: **Davis, Harry R.**
**64 River Bend Road**
**Berkeley Heights, New Jersey 07922(US)**
Inventor: **Sybertz, Edmund J.**
**R.D. 2 10 Ryan Court**
**Chester, New Jersey 07930(US)**

(74) Representative: **von Kreisler, Alek et al**
**Patentanwälte von Kreisler Selting Werner**
**Deichmannhaus am Hauptbahnhof**
**W-5000 Köln 1 (DE)**

## (54) Pharmaceutical compositions comprising natriuretic peptides or neutral endopeptidase inhibitors for treating and preventing myointimal proliferation.

(57) Treatment or prevention of myointimal proliferation in atherosclerosis, restenosis induced by angioplasty or vascular reconstructive surgery, glomerulonephritis, glomerulosclerosis or other diseases involving vascular cell proliferation, with a natriuretic peptide or a neutral endopeptidase inhibitor such as N-[N-[(L)-[1-[(2,2-dimethyl-1,3-dioxolan-4-yl)methoxy]carbonyl]-2-phenylethyl]-L-phenylalanyl]-$\beta$-alanine and N-[2-mercaptomethyl-3-(2-methylphenyl)propionyl]-methionine, or with a neutral endopeptidase inhibitor in combination with a natriuretic peptide or an angiotensin converting enzyme inhibitor are disclosed.

EP 0 533 084 A1

## BACKGROUND OF THE INVENTION

The present invention relates to the treatment and prevention of myointimal proliferation in atherosclerosis, restenosis induced by angioplasty or vascular reconstructive surgery, glomerulonephritis, glomerulosclerosis or other diseases involving vascular cell proliferation, comprising administration of a natriuretic peptide, a neutral endopeptidase (NEP) inhibitor, a combination of a natriuretic peptide and an NEP inhibitor, or a combination of an NEP inhibitor and an angiotensin converting enzyme (ACE) inhibitor.

The invention also relates to a pharmaceutical composition for use in treating or preventing myointimal proliferation in atherosclerosis, restenosis induced by angioplasty or vascular reconstructive surgery, glomerulonephritis, glomerulosclerosis or other diseases involving vascular cell proliferation comprising a natriuretic peptide, an NEP inhibitor, a combination of a natriuretic peptide and an NEP inhibitor, or a combination of an NEP inhibitor and an ACE inhibitor in a pharmaceutically acceptable carrier.

A hallmark of atherosclerosis and restenosis following vascular surgery and angioplasty is smooth muscle cell (SMC) proliferation. See, Ross, "The pathogenesis of atherosclerosis - an update" in N. Eng. J. Med., 8 (1986) 488-500, and Liu, et al., "Restenosis after coronary angioplasty. Potential biological determinants and role of intimal hyperplasia" in Circulation, 79 (1989) 1374-1387. During atherogenesis smooth muscle cells migrate from the media of the artery into the intima, where they proliferate and produce extracellular matrix. This process leads to the encroachment of the arterial lumen which eventually causes the clinical events of atherosclerosis (i.e. myocardial infarct, stroke, limb loss, etc.). Following angioplasty and vascular surgery this process is accelerated and results in restenosis and closure of the arterial segment primarily due to myointimal proliferation.

The term "natriuretic peptides" represents a series of peptide families responsible for fluid homeostasis and blood pressure control. Three types of natriuretic peptides have been identified to date, including atrial natriuretic peptides (ANP), brain natriuretic peptides (BNP) and C-type natriuretic peptides (CNP).

ANP, for example, are a family of vasodilator, diuretic and antihypertensive peptides which maintain salt and water homeostasis as well as to regulate blood pressure. (See, Espiner, et al., Lancet, 1 (1989) 707-710, and Needleman, et al., Ann. Rev. Pharma. Tox., 29 (1989) 23-54). ANP is rapidly inactivated in the circulation by at least two processes: a receptor mediated clearance as reported in Amer. J. Physiol., 256 - (1989) R469-R475, and an enzymatic inactivation via NEP reported in Biochem. J., 243 (1987) 183-187. In addition, ANP stimulates smooth muscle cell particulate guanylate cyclase activity, which results in an increase in the intracellular pool of cyclic guanosine monophosphate (cGMP). It has been reported that cGMP elevating agents inhibit the proliferation of SMCs, in vitro, and that high concentrations of ANP inhibit SMC proliferation in vitro when either platelet derived growth factor (PDGF) or serum are used as mitogens. See, Atherosclerosis, 80 (1989) 143-147, and Biochem. Biophys. Res. Commun., 160 (1989) 1392-1396.

Neutral endopeptidase (EC 3.4.24.11; enkephalinase; atriopeptidase; NEP) is a zinc containing metalloprotease which cleaves a variety of peptide substrates on the amino terminal side of aromatic amino acids. See Biochemistry J., 241 (1987) 237-247. Substrates for this enzyme include, but are not limited to ANP, BNP, CNP, met and leu enkephalin, bradykinin, neurokinin A, and substance P. It has been demonstrated that inhibitors of NEP potentiate the hypotensive, diuretic, natriuretic and plasma ANP responses to pharmacological injection of ANP in experimental animals. The potentiation of ANP by two specific NEP inhibitors is reported by Sybertz, et al. in J. Pharmacol. Ex. Ther., 250 (2), (1990) 624-631, and in Hypertension, 15 (2), (1990) 152-161, while the potentiation of ANP by NEP inhibitors in general was disclosed in U.S. Patent 4,749,688. In U.S. 4,740,499, Olins disclosed the use of thiorphan and kelatorphan to potentiate atrial peptides. Moreover, NEP inhibitors lower blood pressure and exert ANP like effects such as diuresis and increased cGMP excretion in some forms of experimental hypertension. The antihypertensive action of NEP inhibitors is mediated through ANP because antibodies to ANP will neutralize the reduction in blood pressure. However, NEP inhibition is associated with little or no elevation of plasma ANP. See, Sybertz, et al., J. Hypertension, 8 (1990) S161-S167.

ACE inhibitors are a class of drugs known to be effective in treating some types of hypertension by blocking the rise in blood pressure caused by increases in vascular resistance and fluid volume due to the formation of angiotensin II from angiotensin I. The use of ACE inhibitors for treating or preventing restenosis following angioplasty has been claimed, for example in European Patent Application 459,318, but others report that while ACE inhibitors appear to be active against restenosis in rats, studies in primates have not been sucessful.

## SUMMARY OF THE INVENTION

The present invention relates to a method of treating or preventing myointimal proliferation in atherosclerosis, restenosis induced by angioplasty or vascular reconstructive surgery, glomerulonephritis, glomerulosclerosis or other diseases involving vascular cell proliferation, comprising administering an effective amount of a natriuretic peptide or an NEP inhibitor to a mammal in need of such treatment. The method also relates to treating or preventing said diseases or conditions by administering a combination of an ACE inhibitor and an NEP inhibitor or a combination of a natriuretic peptide and an NEP inhibitor.

Another aspect of the present invention relates to pharmaceutical compositions comprising an amount of a natriuretic peptide or an NEP inhibitor effective to treat or prevent myointimal proliferation in atherosclerosis, restenosis induced by angioplasty or vascular reconstructive surgery, glomerulonephritis, glomerulosclerosis or other diseases involving vascular cell proliferation, in a pharmaceutically acceptable carrier. The invention also relates to a pharmaceutical composition comprising a combination of an ACE inhibitor and an NEP inhibitor and a pharmaceutical composition comprising a combination of a natriuretic peptide and an NEP inhibitor in a pharmaceutically acceptable carrier.

## DETAILED DESCRIPTION

The NEP inhibitors suitable for use in this invention include carboxyalkyl dipeptides disclosed in U.S. patent 4,610,816, herein incorporated by reference, having the formula

$R_1{}^aCH(COR_2)\text{-}NH\text{-}CHR_3{}^a\text{-}CONH(CH_2)_p{}^a\text{-}C(R_4{}^aR_5{}^a)\text{-}COR_6{}^a$

wherein:

$R_1{}^a$ is alkyl having from 1 to 6 carbon atoms, adamantylmethyl, cycloalkylmethyl having from 4 to 8 carbon atoms or $A^a\text{-}X^a{}_m{}^a\text{-}C_n{}^aH_{2n}{}^a\text{-}$ {wherein $X^a$ is oxygen or sulfur; $A^a$ is phenyl which may be substituted with the group $Y^a$ (wherein $Y^a$ is halogen, hydroxy, trifluoromethyl, alkoxy having from 1 to 6 carbon atoms, 2- or 3-furanyl, 2- or 3-thienyl, or phenyl (which may be substituted with halogen, hydroxy, trifluoromethyl, alkoxy having from 1 to 6 carbon atoms or alkyl having from 1 to 6 carbon atoms)), benzyl (the phenyl ring of which may be substituted with the group $Y^a$ as defined herein), 1- or 2-naphthyl, 2- or 3-furanyl or 2- or 3-thienyl; $m^a$ is 0 or 1; and $n^a$ is 0, 1, 2, 3 or 4};

$R_2{}^a$ and $R_6{}^a$ may be the same or different and each is independently selected from hydroxy, alkoxy having from 1 to 8 carbon atoms, $B^a\text{-}X^a{}_m{}^a\text{-}C_n{}^aH_{2n}{}^a\text{-}O\text{-}$ {wherein $B^a$ is phenyl (which may be substituted with the group $Y^a$ as defined herein) or 1- and 2-naphthyl; and $X^a$, $m^a$ and $n^a$ are as defined herein provided that, when $n^a = 0$, $m^a$ is zero}, $-OCH_2OCO$-alkyl having from 3 to 8 carbon atoms, $-OCH_2CO$-phenyl {the phenyl ring of which may be substituted with the group $Y^a$ as defined herein), 1-glyceryl,

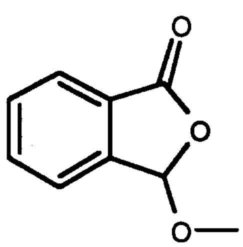

or

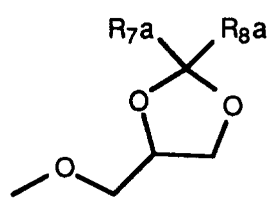

{wherein $R_7{}^a$ is hydrogen, alkyl having from 1 to 6 carbon atoms, or phenyl which may be sustituted with the group $Y^a$ as defined herein, and $R_8{}^a$ is hydrogen or alkyl having from 1 to 6 carbon atoms};

$R_2{}^a$ may also be $-NR_7{}^aR_8{}^a$ wherein $R_7{}^a$ and $R_8{}^a$ are as defined herein;

$R_3{}^a$ is alkyl having from 1 to 6 carbon atoms, cycloalkylmethyl having from 4 to 8 carbon atoms, 2- or 3-thienylmethyl, 2- or 3-furanylmethyl, 1- or 2-naphthylmethyl, or benzyl the phenyl ring of which may be substituted with the group $Y^a$ as defined herein;

$R_4{}^a$ is $D^a\text{-}C_n{}^aH_{2n}{}^a\text{-}O_m{}^a\text{-}$ wherein $D^a$ is selected from hydrogen, alkyl having from 1 to 4 carbon atoms or phenyl which may be substituted with the group $Z^a$; {wherein $Z^a$ is

halogen, hydroxy, trifluoromethyl, alkoxy having from 1 to 6 carbon atoms, or alkyl having from 1 to 6 carbon atoms}; and $m^a$ and $n^a$ are as defined herein;

$R_4{}^a$ may also be $-NR_5{}^aCOR_7{}^a$ {wherein $R_5{}^a$ and $R_7{}^a$ are as defined herein}, or $-NR_5{}^aCO_2R_9{}^a$ {wherein $R_5{}^a$ is as defined herein and $R_9{}^a$ is alkyl having from 1 to 6 carbon atoms or phenyl which may be substituted with the group $Y^a$ as defined herein};

$R_5{}^a$ is hydrogen or alkyl having from 1 to 6 carbon atoms; and

p is 1 or 2;

or pharmaceutically acceptable addition salts thereof; wherein preferred compounds are N-[N-[(L)-[1-[(2,2-di-methyl-1,3-dioxolan-4-yl)-methoxy]carbonyl]-2-phenylethyl]-L-phenyl-alanyl]-β-alanine and N-[N-[(L)-1-carboxy-2-phenylethyl]-L-phenyl-alanyl]-β-alanine;

mercaptoacyl amino acids disclosed in U.S. patent 4,801,609, herein incorporated by reference, having the formulae

$Q^bS\text{-}CH_2\text{-}CH(\text{-}(CH_2)_n{}^b\text{-}R^{1b})\text{-}C(O)\text{-}NH\text{-}CH(R^{2b})\text{-}C(O)\text{-}R^{3b}$ Ib

$Q^bS\text{-}CH_2\text{-}CH(\text{-}(CH_2)_n{}^b\text{-}R^{1ab})\text{-}C(O)\text{-}NH\text{-}CH(R^{2ab})\text{-}C(O)\text{-}R^{3b}$ IIb

$Q^bS\text{-}CH_2\text{-}CH(\text{-}(CH_2)_n{}^b\text{-}R^{1ab})\text{-}C(O)\text{-}NH\text{-}CH(R^{2b})\text{-}C(O)\text{-}R^{3ab}$ IIIb

wherein:

$R^{1b}$ is phenyl substituted by 1 to 3 substituents independently selected from alkyl, alkoxy, cycloalkyl, cyano, and aminomethyl, $y^b\text{-}C_6H_4S\text{-}$; $y^b\text{-}C_6H_4O\text{-}$,

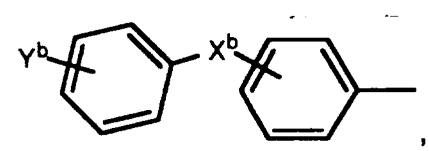

,

a-naphthyl, b-naphthyl, $H_2N(CH_2)m^a\text{-}$ or diphenylmethyl;

$R^{2b}$ is alkyl, alkyl-$S(O)_{0-2}(CH_2)_q{}^b$-, $R^{5b}(CH_2)_k{}^b\text{-}S(O)_{0-2}(CH_2)_q{}^b$-,alkyl-$O(CH_2)_q{}^b$-, $R^{5b}$-$(CH_2)_k{}^b\text{-}O(CH_2)_q{}^b$-, $R^{5b}(CH_2)_q{}^b$-, $H_2N(CH_2)_q{}^b$-, cycloalkyl$(CH_2)_k{}^b$-, $R^{13b}$CONH-$(CH_2)_q{}^b$-, $R^{13b}NHCO(CH_2)_q{}^b$- or $R^{6b}OCO(CH_2)_q{}^b$-;

$R^{3b}$ is $-OR^{7b}$, $-NR^{7b}R^{8b}$,

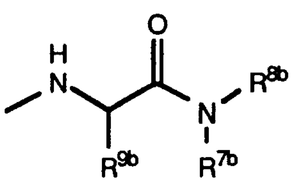

or

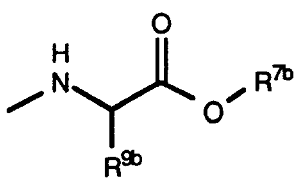

;

$R^{4b}$ and $R^{13b}$ are independently hydrogen, alkyl or $Y^{1b}\text{-}C_6H_4$-;

$R^{5b}$ is $Y^{2b}\text{-}C_6H_4$-, $Y^{2b}\text{-}C_6H_4S$-, $Y^{2b}\text{-}C_6H_4O$-, a-naphthyl, b-naphthyl or

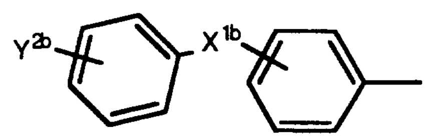

provided that when $R^{5b}$ is $Y^{2b}\text{-}C_6H_4S$- or $Y^{2b}\text{-}C_6H_4O$-, $k^b$ is 2 or 3;

$R^{6b}$, $R^{7b}$ and $R^{8b}$ are independently H, alkyl, hydroxyalkyl, alkoxyalkyl, aminoalkyl, alkylaminoalkyl, dialkylaminoalkyl, phenylalkyl, naphthylalkyl, $Y^b$-phenylalkyl or $Y^b$-naphthylalkyl;

$R^{9b}$ is hydrogen alkyl, carboxyalkyl, mercaptoalkyl, alkylthioalkyl, aminoalkyl, hydroxyalkyl, phenylalkyl, hydroxyphenylalkyl, guanidinoalkyl or carbamoylalkyl;

$n^b$ is 0-2;

$m^b$ and $k^b$ are independently 0-3;
$q^b$ is 1-4;
$X^b$ and $X^{1b}$ are independently a bond, -O-, -S- or $-CH_2-$;
$Q^b$ is hydrogen or $R^{10b}CO-$;
$R^{10b}$ is alkyl, hydroxyalkyl, alkoxyalkyl, dialkylaminoalkyl, $Y^{3b}-C_6H_4$-alkyl, alkoxy, $Y^{3b}-C_6H_4-$ or naphthyl;
$Y^b$, $Y^{1b}$, $Y^{3b}$ and $Y^{3b}$ independently represent 1 to 3 substituents selected from H, alkyl, cycloalkyl, alkoxy, OH, F, Cl, Br, CN, $-CH_2NH_2$, -COOH, -COOalkyl, $-CONH_2$ and phenyl;
$R^{1ab}$ is $Y^b-C_6H_4-$, $Y^b-C_6H_4S-$, $Y_b-C_6H_4O-$,

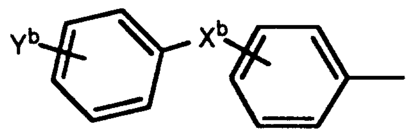

,

a-naphthyl, b-naphthyl, $H_2N(CH_2)_m{}^b-$ or diphenylmethyl;
$R^{2ab}$ is $R^{5ab}(CH)_k{}^bS(O)_{0-2}(CH_2)_q{}^b-$, $R^{5ab}(CH)_q{}^b-$ or cycloalkyl-$(CH_2)_k{}^b-$, and when $R^{3b}$ is $-NR^{7b}R^{8b}$,

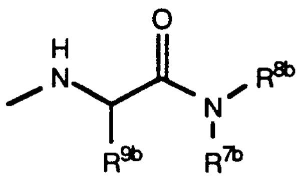

or

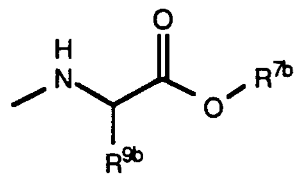

,

$R^{2ab}$ may also be $R^{13b}CONH(CH_2)_q{}^b-$, $R^{13b}NHCO(CH_2)_q{}^b-$or $R^{6b}OCO(CH_2)_q{}^b-$;
$R^{3ab}$ is $-OR^{11b}$, $-NR^{11b}R^{12b}$,

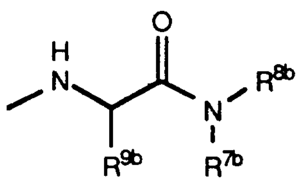

or

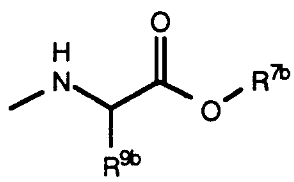

;

$R^{5ab}$ is $Y^{3b}-C_6H_4-$ provided $Y^{3b}$ is not H or OH, $Y^{3b}-C_6H_4S-$, $Y^{3b}-C_6H_4O-$, a-naphthyl, b-naphthyl or

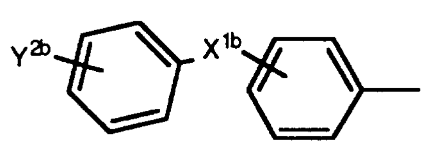

,

provided that when $R^{5ab}$ is $Y^{3b}-C_6H_4S-$, $Y^{3b}-C_6H_4O-$, $k^b$ is 2 or 3;
$R^{11b}$ is hydroxyalkyl, substituted phenylalkyl wherein the phenyl group is substituted by 1 or more groups selected from alkyl, alkoxy, cycloalkyl and cyano;
$R^{12b}$ is H or selected from the same group as $R^{11b}$;

wherein the term alkyl or each alkyl portion has 1-6 carbon atoms and cycloalkyl is $C_3-C_6$; or pharmaceutically acceptable addition salts thereof;

mercaptoacyl amino acids disclosed in U.S. patent 4,929,641, herein incorporated by reference, having the formula

$Q^cS\text{-}CH_2\text{-}CH(\text{-}(CH_2)_{n^c}\text{-}R^{1c})\text{-}C(O)\text{-}NH\text{-}CH(R^{2c})\text{-}C(O)\text{-}R^{3c}$

wherein $R^{1c}$ is phenyl substituted by alkyl, $R^{2c}$ is alkyl-$S(O)_{0-2}(CH_2)_q{}^c$-, $R^{3c}$ is $OR^{7c}$ wherein $R^{7c}$ is hydrogen or lower alkyl, $Q^c$ is hydrogen or $R^{10c}$CO- wherein $R^{10c}$ is alkyl, $n^c$ is 0-2 and $q^c$ is 1-4; wherein preferred compounds are N-[2-acetylthiomethyl-3-(2-methylphenyl)propionyl]-methionine ethyl ester and N-[2-mercapto-methyl-3-(2-methylphenyl)propionyl]-methionine;

mercaptoacyl amino acids disclosed in PCT Publication WO90/12003, having the formula

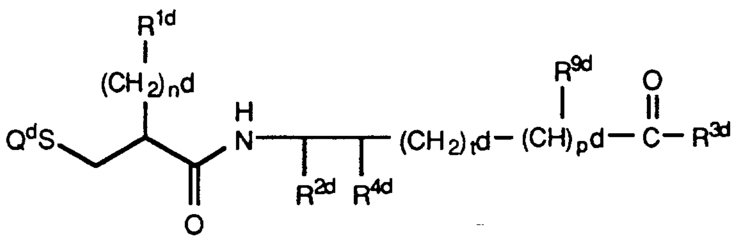

wherein:

| | |
|---|---|
| $Q^d$ | is hydrogen or $R^{7d}$CO-; |
| $R^{1d}$ | is lower alkyl, cyclolower alkyl, aryl or heteroaryl; |
| $R^{2d}$ | is hydrogen; lower alkyl; cyclolower alkyl; lower alkyl substituted with hydroxy, lower alkoxy mercapto, lower alkylthio, aryl or heteroaryl; aryl; or heteroaryl; |
| $R^{3d}$ | is $-OR^{5d}$ or $-NR^{5d}R^{6d}$; |
| $R^{4d}$ and $R^{9d}$ | are independently $-(CH_2)_q{}^dR^{8d}$, provided that when $R^{4d}$ and $R^{9d}$ are both hydrogen, $R^{2d}$ is biphenyl, phenoxyphenyl, phenylthiophenyl, naphthyl, heteroaryl or lower alkyl substituted with hydroxy, lower alkoxy, mercapto or lower alkylthio; |
| $R^{5d}$ and $R^{6d}$ | are independently selected from the group consisting of hydrogen, lower alkyl, hydroxy lower alkyl, lower alkoxy lower alkyl and aryl lower alkyl, or $R^{5d}$ and $R^{6d}$ together with the nitrogen to which they are attached form a 5-7 membered ring; |
| $R^{7d}$ | is hydrogen, lower alkyl or aryl; |
| $R^{8d}$ | is hydrogen, hydroxy, lower alkoxy, mercapto, lower alkylthio, aryl or heteroaryl; |
| $n^d$ | is 1 or 2; |
| $p^d$ | is 0 or 1 |
| $q^d$ | is 0,1 or 2; |
| $t^d$ | is 0 or 1; |

and pharmaceutically acceptable salts thereof; wherein preferred compounds are N-[2(S)-mercapto-methyl-3-(2-methylphenyl)-propanoyl]-(S)-isoserine and N-(S)-[3-mercapto-2-(2-methylphenyl)-propionyl]-(S)-2-methoxy-$\beta$-alanine;

carboxyalkyl dipeptides disclosed in PCT Publication WO91/05796, having the formula

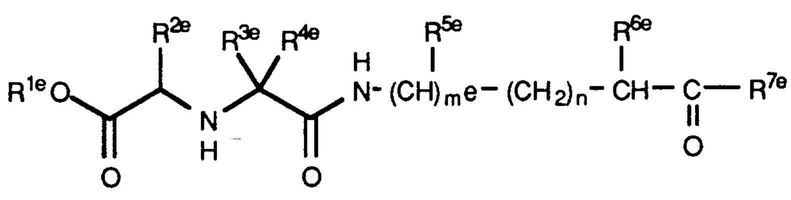

wherein:

| | |
|---|---|
| $R^{1e}$ | is H, alkyl, arylalkyl, or aryl; |
| $R^{2e}$ | is H, alkyl, alkenyl or alkynyl, wherein the alkyl portion is substituted with 0-3 substituents independently selected from the group consisting of hydroxy, alkoxy, alkoxyalkoxy, alkylthio, aryl, alkoxyalkylthio, arylalkoxy and arylalkylthio; |
| $R^{3e}$ and $R^{4e}$ | are independently alkyl or arylalkyl; or $R^{3e}$ and $R^{4e}$ together with the carbon to which they are attached form a 5-, 6- or 7-membered ring wherein said ring comprises 0 to 1 heteroatoms selected from the group consisting of sulfur and oxygen, wherein said ring |

| | |
|---|---|
| | is unsubstituted or is substituted on a carbon atom ring member by an alkyl or aryl group, or wherein said ring is substituted by a fused benzene ring; |
| $R^{5e}$ | is H, alkyl, alkoxyalkyl, alkylthioalkyl, aryl, arylalkyl, heteroaryl, heteroarylalkyl, arylalkoxyalkyl or arylalkylthioalkyl; |
| $R^{6e}$ | is H, hydroxy, alkoxy, alkyl, alkoxyalkyl, alkylthioalkyl, arylalkoxyalkyl, arylalkylthioalkyl, aryl, heteroaryl, arylalkyl or heteroarylalkyl; |
| $R^{7e}$ | is hydroxy, alkoxy, aryloxy, arylalkoxy, amino, alkylamino or dialkylamino; |
| $m^e$ | is 0 or 1; |
| $n^e$ | is 0, 1, 2 or 3; |

or a pharmaceutically acceptable salt thereof; wherein preferred compounds are N-[1-[[1(S)-benzyloxycarbonyl-3-phenylpropyl]amino]cyclopentylcarbonyl]-(S)-isoserine and N-[1-[[1(S)-carbonyl-3-phenylpropyl]-amino]-cyclopentylcarbonyl]-(S)-isoserine;

disulfide derivatives of mercaptoacyl amino acids disclosed in PCT Publication WO91/17980, having the formulae

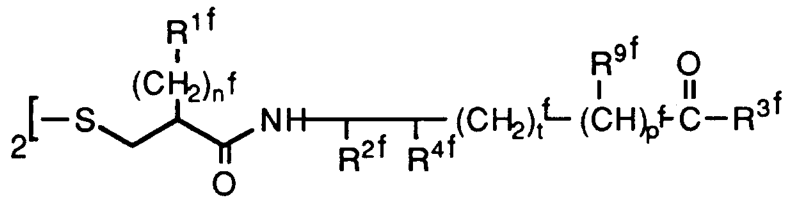

If

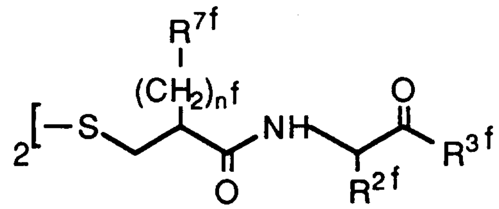

IIf

wherein:

| | |
|---|---|
| $R^{1f}$ | is lower alkyl, cyclolower alkyl, aryl or heteroaryl; |
| $R^{2f}$ | is hydrogen; lower alkyl; cyclolower alkyl; lower alkyl substituted with hydroxy, lower alkoxy, mercapto, lower alkylthio, aryl, heteroaryl, aralkyloxy or aralkylthio; aryl; or heteroaryl; |
| $R^{3f}$ | is $-OR^{5f}$ or $-NR^{5f}R^{6f}$; |
| $R^{4f}$ and $R^{9f}$ | are independently $-(CH_2)_q{}^fR^{8f}$; |
| $R^{5f}$ and $R^{6f}$ | are independently selected from the group consisting of hydrogen, lower alkyl, hydroxy lower alkyl, lower alkoxy lower alkyl and aryl lower alkyl, or $R^{5f}$ and $R^{6f}$ together with the nitrogen to shich they are attached form a 5-7 membered ring; |
| $R^{7f}$ | is phenyl substituted by 1 to 3 substituents selected from the group consisting of lower alkyl, lower alkoxy, cycloalkyl, halo, cyano and aminomethyl; |
| $R^{8f}$ | is hydrogen, hydroxy, lower alkoxy, mercapto, lower alkylthio, aryl or heteroaryl; |
| $n^f$ | is 1 or 2; |
| $p^f$ | is 0 or 1; |
| $q^f$ | is 0, 1, or 2; |
| $t^f$ | is 0 or 1; |

or a pharmaceutically acceptable salt thereof; wherein preferred compounds are 1,1'-[dithiobis-[2(S)-(2-methylbenzyl)-1-oxo-3,1-propanediyl]]-bis-(S)-isoserine and 1,1'-[dithio-bis-[2(S)-(2-methylbenzyl)-1-oxo-3,1-propanediyl]]-bis-(S)-methionine;

mercaptoacyl amino acids disclosed in PCT publication WO90/02117, having the formula

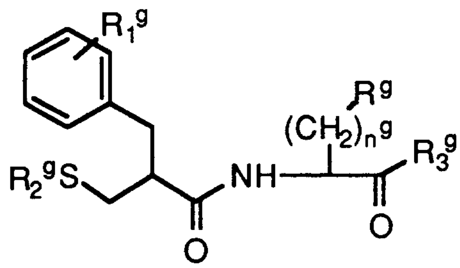

wherein:

$R^g$ is a group of the formulae

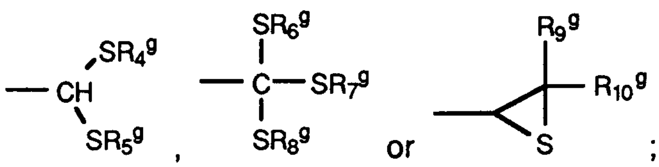

, or ;

$R_1^g$ is 1-3 substituents selected from the group consisting of hydrogen, halogen, lower alkyl, cycloalkyl, lower alkoxy, OH, aryl, aryloxy, CN, $-CH_2NH_2$, -COOH, lower alkoxycarbonyl and $-CONH_2$;

$R_2^g$ is H, $-COR_{11}^g$ or $-CH_2OCOR_{11}^g$;

$R_3^g$ is $-OR_{12}^g$, $-NR_{12}^gR_{13}^g$ or $-OCH(R_{14}^g)CONR_{12}^gR_{13}^g$;

$R_4^g$, $R_5^g$, $R_6^g$, $R_7^g$, $R_8^g$, $R_9^g$ and $R_{10}^g$ are each independently lower alkyl; or aralkyl or cycloalkylalkyl, wherein the aryl or cycloalkyl ring is optionally substituted by 1-3 substituents selected from lower alkyl, OH, halogen, lower alkoxy and $NH_2$; or $R_4^g$ and $R_5^g$ together with the carbon to which they are attached comprise a 5-, 6- or 7-membered ring;

$R_{11}^g$ is lower alkyl, aryl or arylmethyl;

$R_{12}^g$ and $R_{13}^g$ are each independently hydrogen; lower alkyl, optionally substituted by 1-2 OH or lower alkoxy groups; lower alkoxyalkoxy; halogen; lower haloalkoxy; $NH_2$; alkylamino; dialkylamino; Ar; or Het; or $R_{12}^g$ and $R_{13}^g$ together with the nitrogen to which they are attached comprise a 5-, 6- or7-membered ring, wherein said ring may contain ring members selected from the group consisting of -N-, -N(alkyl)- or -O-, and wherein said ring is optionally substituted at a carbon atom ring member by an alkyl or OH substituent;

$Ar^g$ is an aryl group optionally substituted by 1-3 substituents selected from the group consisiting of lower alkyl, OH, halogen, lower alkoxy and $NH_2$;

$Het^g$ is a 5- or 6-membered saturated ring, wherein 1-2 ring memebers are oxygen atoms and wherein said ring is optionally substituted by 1-2 lower alkyl groups;

$R_{14}^g$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl or carbamoylalkyl;

$n^g$ is 0, 1, 2, 3 or 4;

and pharmaceutically acceptable addition salts thereof; wherein a preferred compound is N-(3-phenyl-2-(mercaptomethyl)-propionyl)-(S)-4-(methylmercapto)methionine;

mercaptoacyl amino acids disclosed in U.S. patent 4,879,309, incorporated herein by reference, having the formula

$R_1^hS-CH_2-CH(-(CH_2)_n^h-R_2^h)-C(O)-NR_4^h-A^h-C(O)-R_3^h$

wherein:

$R_1h$ is H or $R_5{}^hCO$-;

$R_2{}^h$ is $Y^h$-$C_6H_4$-, $Y^h$-$C_6H_4S$-, $Y^h$-$C_6H_4O$-, $Y^h$-$C_6H_4CH_2S$-,$Y^h$-$C_6H_4CH_2O$-, a-naphthyl, b-naphthyl, fury, thienyl, benzofuryl, benzothienyl, diphenylmethyl or

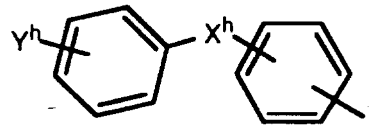

;

$R_3{}^h$ is -$OR_6h$, -$NR_6{}^hR_7{}^h$ or

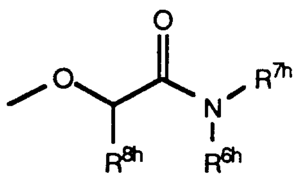

;

$R_4{}^h$ is hydrogen, lower alkyl or aryl lower alkyl;

$R_5{}^h$ is lower alkyl, hydroxylower alkyl, lower alkoxy lower alkyl, (di-lower alkyl) amino lower alkyl, $Y_1{}^h$-$C_6H_4$-lower alkyl, lower alkoxy, $Y_1{}^h$-$C_6H_4$-, naphthyl, furyl, thienyl or pyridyl;

$R_6{}^h$ and $R_7{}^h$ are independently hydrogen, lower alkyl or substituted lower alkyl wherein the substituents are selected from the group consisting of 1 or 2 hydroxy groups, 1 or 2 lower alkoxy groups, lower alkoxy lower alkoxy, halogeno, halogeno lower alkoxy, amino, mono- or di-lower alkyl amino, heterocycloalkyl, lower alkyl heterocycloalkyl, aryl, substituted aryl wherein the substituents on aryl are 1-3 substituents selected from the group consisting of lower alkyl, hydroxy, halogeno, lower alkoxy and amino, and a 5-6 membered saturated ring comprising 1-2 oxygen atoms as ring members wherein the ring carbon atoms are substituted with 0-2 lower alkyl substituents; or $R_6{}^h$ and $R_7{}^h$ together with the nitrogen to which they are attached complete a 5-7 membered ring, wherein 0-1 of the 4-6 ring members comprising $R_6{}^h$ and $R_7{}^h$ is a nitrogen atom, an alkyl substituted nitrogen atom or an oxygen atom, and wherein the ring is substituted on the ring carbon atoms with 0-3 substituents selected from the group consisting of alkyl and hydroxy;

$R_8{}^h$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl or carbamoylalkyl;

$n^h$ is 0-3;

$A^h$ is

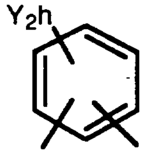 , 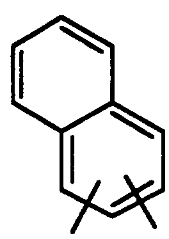 , 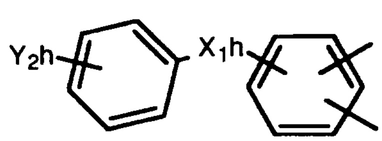 or 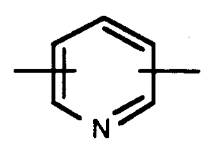 ;

$X^h$ and $X_1{}^h$ are independently a bond, -O-, -S- or -$CH_2$-;

$Y^h$, $Y_1{}^h$ and $Y_2{}^h$ are independently 1 to 3 substituents selected from the group consisting of hydrogen, lower alkyl, cyclolower alkyl, lower alkoxy, OH, F, Cl, Br, I, -CN, -COOH,

-COO-lower alkyl, $-CH_2NH_2$, $-CONH_2$ and aryl;

and the pharmaceutically acceptable acid addition salts thereof; wherein preferred compounds are N-[2-acetylthiomethyl-3-phenylpropionyl]-3-aminobenzoic acid and N-[2-mercaptomethyl-3-phenylpropionyl]-3-aminobenzoic acid;

carboxyalkylcarbonyl amino acids disclosed in PCT Publication WO91/07386, having the formula

$R^{1i}O\text{-}C(O)\text{-}CH(R^{2i})\text{-}CH_2\text{-}C(R^{3i}R^{4i})\text{-}C(O)\text{-}NH\text{-}(CHR^{5i})_m{}^{i}\text{-}(CH_2)_n{}^{i}\text{-}CH(R^{6i})\text{-}C(O)\text{-}R^{7i}$

wherein:

- $R^{1i}$ is H, alkyl, arylalkyl, aryl or aryloxyalkyl;
- $R^{2i}$ is alkyl, alkenyl, alkynyl, alkoxy or alkylthio, wherein the alkyl portion is substituted with 0-3 substituents independently selected from the group consisting of hydroxy, alkoxy, alkoxyalkoxy, alkylthio, aryl, alkoxyalkylthio, arylalkoxy and arylalkylthio;
- $R^{3i}$ and $R^{4i}$ are independently alkyl or arylalkyl; or $R^{3i}$ and $R^{4i}$ together with the carbon to which they are attached form a 5-, 6- or 7-membered ring wherein said ring comprises 0 to 1 heteroatoms selected from the group consisting of sulfur and oxygen, wherein said ring is unsubstituted or is substituted on a carbon atom ring member by an alkyl or aryl group, or wherein said ring is substituted by a fused benzene ring;
- $R^{5i}$ is H, alkyl, alkoxyalkyl, alkylthioalkyl, aryl, arylalkyl, heteroaryl, heteroarylalkyl, arylalkoxyalkyl or arylalkylthioalkyl;
- $R^{6i}$ is H, hydroxy, alkoxy, alkyl, arylalkoxy, alkoxyalkyl, alkylthioalkyl, arylalkoxyalkyl, arylalkylthioalkyl, aryl or heteroaryl;
- $R^{7i}$ is hydroxy, alkoxy, aryloxy, arylalkoxy, amino, alkylamino or dialkylamino;
- $m^i$ is 0 or 1;
- $n^i$ is 0, 1, 2 or 3;

or a pharmaceutically acceptable salt thereof; wherein a preferred compound is N-[1-(2-carboxy-4-phenylbutyl)-cyclopentanecarbonyl]-(S)-isoserine;

mercaptocycloalkyl amino acids disclosed in PCT Publication WO91/09840, having the formula

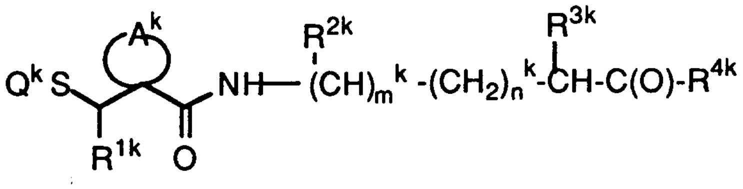

wherein:

- $A^k$ is a 4-, 5- or 6-membered alkylene chain substituted with 1 to 3 substituents selected from the group consisting of hydrogen, hydroxy, alkyl or aryl; a 4-, 5- or 6-membered alkenylene chain, wherein 1 to 3 of the saturated carbon atoms are substituted as defined for the alkylene chain; a 4-, 5- or 6-membered hetero-atom-containing chain comprising 2 to 5 carbon atoms and 1 or 2 hetero atoms selected from the group consisting of oxygen and sulfur, wherein when 2 heteroatoms are present, the heteroatoms are non-adjacent; or an alkylene or hetero-atom-containing chain as defined above wherein said alkylene or hetero-atom-containing chain is substituted with a fused benzene ring;
- $Q^k$ is hydrogen or $R^{5k}CO$-;
- $R^{1k}$ is H, alkyl, arylalkyl or aryl;
- $R^{2k}$ is H, alkyl, alkoxyalkyl, alkylthioalkyl, aryl, arylalkyl, heteroaryl, heteroarylalkyl, arylalkoxyalkyl, or arylalkylthioalkyl;
- $R^{3k}$ is H, hydroxy, alkoxy, alkyl, arylalkoxy, alkoxyalkyl, alkylthioalkyl, arylalkoxyalkyl, arylalkylthioalkyl, aryl, heteroaryl, arylalkyl or heteroarylalkyl, or $R^{2k}$ and $R^{3k}$ form an alkylene chain of 1 to 5 carbon atoms, thereby, together with the carbons to which they are attached, completing a 5-, 6-, or 7-membered ring;
- $R^{4k}$ is hydroxy, alkoxy, aryloxy, arylalkoxy, amino, alkylamino or dialkylamino;
- $R^{5k}$ is alkyl or aryl;
- $m^k$ is 0 or 1;
- $n^k$ is 0, 1, 2 or 3;

or a pharmaceutically acceptable salt thereof; wherein a preferred compound is N-[1-(acetylthiomethyl)-cyclopentanecarbonyl]-(S)-methionine ethyl ester;

mercaptoacyl aminolactams disclosed in U.S.Patent 5,075,302, incorporated herein by reference, having the formula

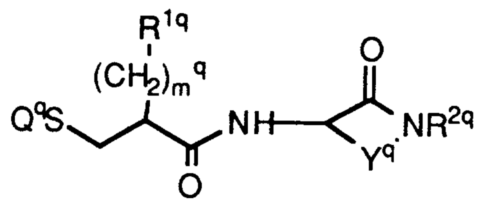

wherein:

| | |
|---|---|
| $Y^q$ | is $-(CHR^{5q})_n{}^q-(CR^{3q}R^{4q})-$ or $-(CR^{3q}R^{4q})_p{}^qX^q(CR^{3q}R^{4q})_q{}^q-$,wherein two substituents selected from the group consisting of $R^{3q}$, $R^{4q}$ and $R^{5q}$, together with the carbons to which the substituents are attached, when the substituents are present on adjacent carbon atoms, can form a benzene, cyclopentane or cyclohexane ring; |
| $X^q$ | is -O-, -S-, -SO- or $-SO_2-$; |
| $Q^q$ | is hydrogen or $R^{6q}CO-$; |
| $m^q$ | is 1 or 2; |
| $n^q$ | is 1, 2, 3 or 4; |
| $p^q$ | is 1 or 2; |
| $q^q$ | is 2 or 3; |
| $R^{1q}$ | is lower alkyl; aryl selected from the group consisting of phenyl, naphthyl, substituted phenyl and substituted naphthyl, wherein said substituted phenyl and substituted naphthyl groups are substituted with 1-3 substituents selected from the group consisting of lower alkyl, hydroxy, lower alkoxy, halo, trifluoromethyl, phenyl, phenoxy, and phenylthio; heteroaryl or substituted heteroaryl, wherein heteroaryl is selected from the group consisting of furanyl, thienyl, pyrrolyl and pyridyl, and wherein said substituted heteroaryl is substituted with 1-3 substituents selected from the group consisting of lower alkyl, hydroxy, lower alkoxy, halo, trifluoromethyl, phenyl, phenoxy, and phenylthio; |
| $R^{2q}$ | is hydrogen, lower alkyl, hydroxylower alkyl, loweralkoxylower alkyl, aryllower alkyl, heteroaryllower alkyl, substituted-aryllower alkyl or substituted-heteroaryllower alkyl, wherein aryl, heteroaryl, substituted aryl and substituted heteroaryl are as defined above; |
| $R^{3q}$ and $R^{4q}$ | are independently hydrogen, loweralkyl, aryllower alkyl, heteroaryllower alkyl, substituted-aryllower alkyl or substituted-heteroaryllower alkyl, wherein aryl, heteroaryl, substituted aryl and substituted heteroaryl are as defined above; |
| $R^{5q}$ | is hydrogen, loweralkyl, aryllower alkyl, heteroaryllower alkyl, hydroxy, lower alkoxy, mercapto, lower alkylthio, substituted-aryllower alkyl or substituted-heteroaryllower alkyl, wherein aryl, heteroaryl, substituted aryl and substituted heteroaryl are as defined above; and |

$R^{6q}$ is lower alkyl, aryl or heteroaryl, wherein aryl and heteroaryl are as defined above; wherein a preferred compound is 3(S)-[2-(acetylthio-methyl)-3-phenylpropionyl]amino-ϵ-caprolactam;

glutaryl amino acids disclosed in U.S. 4,975,444, herein incorporated by reference, having the formula

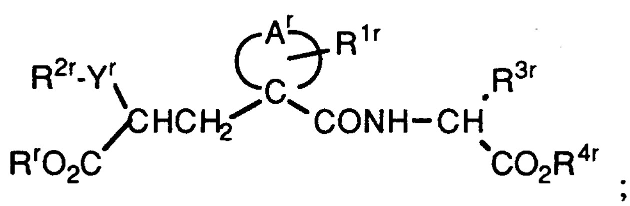

wherein:

| | |
|---|---|
| $A^r$ | completes a 5 or 6 membered carbocyclic ring which may be saturated or monounsaturated; |
| $R^{1r}$ | is H or $(C_1-C_4)$alkyl; |

$R^r$ and $R^{4r}$ are each independently H, $(C_1\text{-}C_6)$alkyl, $(C_3\text{-}C_7)$cycloalkyl, benzyl or an alternative biolabile ester-forming group;

$Y^r$ is either a direct bond or an alkylene group of from 1 to 6 carbon atoms which may be straight or branched chain;

$R^{2r}$ is H, aryl, heterocyclyl, $R^{6r}CONR^{5r}$-, $R^{7r}NR^{5r}CO$-, $R^{7r}NR^{5r}SO_2$- or $R^{8r}SONR^{5r}$-, with the proviso that $Y^r$ is not a direct bond when $R^{2r}$ is H, aryl or heterocyclyl;

$R^{3r}$ is a group of the formula

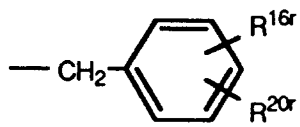

; or

a group of the formula

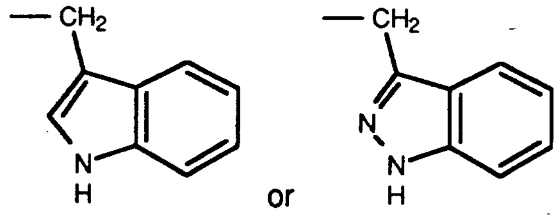

wherein said groups may optionally be substituted in the fused benzene ring by $(C_1\text{-}C_4)$alkyl, $(C_1\text{-}C_4)$alkoxy, OH, halo or $CF_3$;

$R^{5r}$ is H, $(C_1\text{-}C_6)$alkyl or aryl$(C_1\text{-}C_6)$alkyl;

$R^{6r}$ is aryl, heterocyclyl or a group of the formula

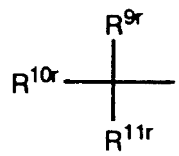

;

$R^{7r}$ is $(C_1\text{-}C_6)$alkyl, aryl, aryl$(C_1\text{-}C_6)$alkyl, heterocyclyl, heterocyclyl$(C_1\text{-}C_6)$alkyl or a group of the formula

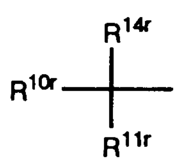

;

$R^{8r}$ is $(C_1\text{-}C_6)$alkyl, aryl, aryl$(C_1\text{-}C_6)$alkyl, heterocyclyl or heterocyclyl$(C_1\text{-}C_6)$alkyl;

$R^{9r}$ is H, OH, $(C_1\text{-}C_6)$alkoxy, $(C_1\text{-}C_6)$alkyl, hydroxy$(C_1\text{-}C_6)$alkyl, aryl, aryl$(C_2\text{-}C_6)$heterocyclyl, heterocyclyl$(C_1\text{-}C_6)$alkyl, $R^{12r}CONH$-, $R^{12r}SO_2NH$- or $(R^{13r})_2N$-;

$R^{10r}$ and $R^{11r}$ are each independently H or alkyl; or $R^{10r}$ is H and $R^{11r}$ is amino, $(C_1\text{-}C_6)$alkyl, imidazolylmethyl, aryl, aryl$(C_1\text{-}C_6)$alkyl, hydroxy$(C_1\text{-}C_6)$alkyl or methylthio$(C_1\text{-}C_6)$alkyl; or the two groups $R^{10r}$ and $R^{11r}$ are joined together to form, with the carbon atom to which they are attached, a 3 to 6 membered carbocyclic ring or a ring which may optionally be substituted by amino, $(C_2\text{-}C_4)$alkanoyl or aroyl; or a pyrrolidine or piperidine ring which is substituted by amino, $(C_2\text{-}C_4)$alkyl or aroyl; amino;

$R^{12r}$ is $(C_1\text{-}C_6)$alkyl, $(C_3\text{-}C_7)$cycloalkyl, aryl, aryl$(C_1\text{-}C_6)$alkyl, heterocyclyl or heterocyclyl$(C_1\text{-}C_6)$alkyl;

$R^{13r}$ is H, $(C_1$-$C_6)$alkyl, aryl or the two groups $R^{13r}$ are taken together to form, with the nitrogen to which they are attached, a pyrrolidinyl, piperidino, morpholino, piperazinyl or N-$(C_1$-$C_6)$alkyl-piperazinyl group;

$R^{14r}$ is $(R^{13r})_2NCO$-, $R^{12r}OCH_2$- or $R^{15r}OCO$-;

$R^{15r}$ is $(C_1$-$C_6)$alkyl, $(C_3$-$C_7)$cycloalkyl or aryl$(C_1$-$C_6)$alkyl;

$R^{16r}$ is H, halo, 4-hydroxy, 4-$(C_1$-$C_6$ alkoxy), 4-$(C_3$-$C_7$ cycloalkoxy), 4-$(C_2$-$C_6$ alkenyloxy), 4-[$(C_1$-$C_6$ alkoxy)carbonyloxy], 4-[$(C_3$-$C_7$ cycloalkoxy)carbonyloxy] or 3-$(C_1$-$C_4$ alkyl)-$SO_2NH$-;

$R^{20r}$ is H, $(C_1$-$C_4)$alkyl, $(C_1$-$C_4)$alkoxy$(C_2$-$C_6)$alkanoyl or halo;

and pharmaceutically acceptable salts thereof and bioprecursors therefor;

glutaryl amino acids disclosed in European Patent Application 274,234, having the formula

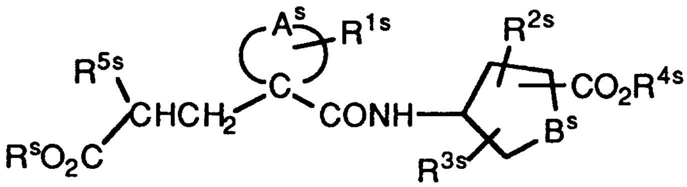

wherein $A^s$ completes a 4 to 7 membered carbocyclic ring which may be saturated or mono-unsaturated and which may optionally be fused to a further saturated or unsaturated 5 or 6 membered carbocyclic ring;

$B^s$ is $(CH_2)m^s$ wherein $m^s$ is an integer of from 1 to 3;

each of $R^s$ and $R^{4s}$ is independently H, alkyl, benzyl or an alternative biolabile ester-forming group;

$R^{1s}$ is H or alkyl;

$R^{2s}$ and $R^{3s}$ are each independently H, OH, alkyl or alkoxy;

and $R^{5s}$ is alkyl, alkenyl, alkynyl, arylalkynyl, cycloalkyl, cycloalkenyl, alkoxy, -$NR^{6s}R^{7s}$, -$NR^{8s}COR^{9s}$, -$NR^{8s}SO_2R^{9s}$ or a saturated heterocyclic group;

or alkyl substituted by one or more substituents chosen from halo, hydroxy, alkoxy, hydroxyalkoxy, alkoxyalkoxy, cycloalkyl, cycloalkenyl, aryl, aryloxy, arlyoxyalkoxy, heterocyclyloxy, -$NR^{6s}R^{7s}$,-$NR^{8s}COR^{9s}$, -$NR^{8s}SO_2R^{9s}$, -$CONR^{6s}R^{7s}$, -SH, $S(O)_{p^s}R^{10s}$, -$COR^{11s}$ or -$CO_2R^{12s}$;

wherein $R^{6s}$ and $R^{7s}$ are each independently H, alkyl, cycloalkyl (optionally substituted by hydroxy or alkoxy), aryl, arylalkyl, alkoxyalkyl or heterocyclyl; or the two groups $R^{6s}$ and $R^{7s}$ are taken together with the nitrogen to which they are attached to form a pyrrolidinyl, piperidino, morpholino, piperazinyl or N-alkyl-piperazinyl group;

$R^{8s}$ is H or alkyl;

$R^{9s}$ is alkyl, $CF_3$, aryl, aryl, alkyl, arylalkoxy, heterocyclyl, alkoxy or -$NR^{6s}R^{7s}$ wherein $R^{6s}$ and $R^{7s}$ are as previously defined;

$R^{10s}$ is alkyl, aryl, heterocyclyl, or -$NR^{6s}R^{7s}$ wherein $R^{6s}$ and $R^{7s}$ are as previously defined;

$R^{11s}$ is alkyl, cycloalkyl, aryl or heterocyclyl;

$R^{12s}$ is H or alkyl;

and $p^s$ is 0,1 or 2;

and pharmaceutically acceptable salts thereof and bioprecursors therefor;

amino acid derivatives disclosed in European Patent Publication 419,327A1, having the formula

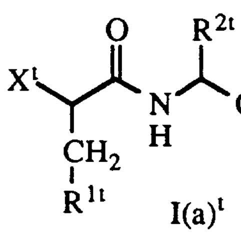 I(a)$^t$ or 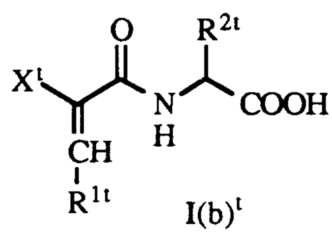 I(b)$^t$

wherein $R^{1t}$ is biphenyl, $Q^t$ or

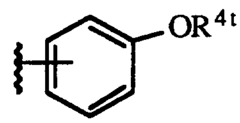

;

$Q^t$ is selected from the group consisting of

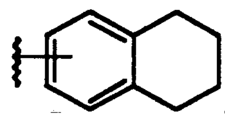

,

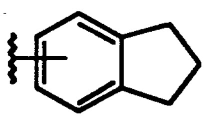

,

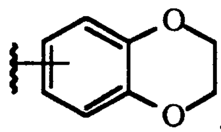

,

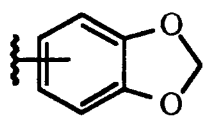

and

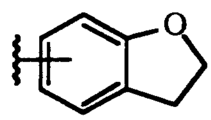

;

$R^{4t}$ is hydrogen, lower alkyl, phenyl or phenyl(lower alkyl);

$R^{2t}$ is selected from the group consisting of hydrogen, lower alkyl, hydroxy(lower alkyl), phenyl, phenyl-(lower alkyl), hydroxyphenyl(lower alkyl), amino(lower alkyl), guanidino(lower alkyl), mercapto(lower alkyl), lower alkylthio(lower alkyl), imidazolyl(lower alkyl), indolyl(lower alkyl), carbamyl(lower alkyl), carboxy(lower alkyl) and $-CH_2-Q^t$, wherein $Q^t$ is as defined above;

$X^t$ is selected from the group consisting of mercaptomethyl, hydroxamic acid, $-NH-CH(R^{3t})-COOH$,

$$-(CH_2)_{mt}-\overset{\overset{\displaystyle O}{\|}}{P}(OH)_2,$$

wherein mt = 0 or 1, and

$$-\overset{H}{N}-\overset{\overset{\displaystyle O}{\|}}{P}(OH)_2;$$

and pharmaceutically acceptable salts thereof and bioprecursors therefor; and

glutaryl amino acids disclosed in European Patent Application 343,911, having the formula

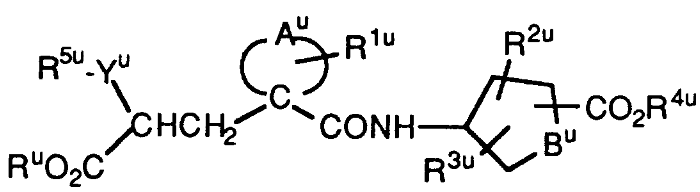

wherein $A^u$ completes a 4 to 7 membered carbocyclic ring which may be saturated or mono-unsaturated and which may optionally be fused to a further saturated or unsaturated 5 or 6 membered carbocyclic ring;

$B^u$ is $(CH_2)m^u$ wherein $m^s$ is an integer of from 1 to 3;

each of $R^u$ and $R^{4u}$ is independently H, alkyl, benzyl or an alternative biolabile ester-forming group;

$R^{1u}$ is H or alkyl;

$R^{2u}$ and $R^{3u}$ are each independently H, OH, alkyl or alkoxy, or $R^{2u}$ and $R^{3u}$ are linked together and are $(CH_2)_r{}^u$ wherein $r^u$ is an integer from 1 to 4;

$Y^u$ is an optional alkylene group of from 1 to 6 carbon atoms which may be straight or branched-chain; and $R^{5u}$ is $R^{6u}CONR^{9u}$-, $R^{6u}SO_2NR^{9u}$-, $R^{6u}CO_2$-, $R^{6u}CO$-, $R^{6u}SO_q{}^u$-, $R^{7u}NR^{9u}SO_2$-, or $R^{7u}OCO$-;
wherein $R^{6u}$ is a group of the formula

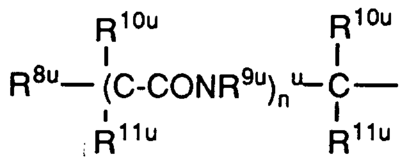

$R^{7u}$ is a group of the formula

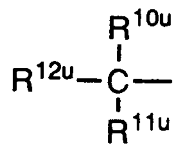

and $R^{9u}$ is H, alkyl, aryl, cycloalkyl, heterocyclyl, arylalkyl, or hererocyclylalkyl;

wherein $R^{8u}$ is $R^{9u}CONR^{9u}$-, $R^{9u}SO_2NR^{9u}$-, $R^{13u}R^{14u}N$-$(CH_2)_p{}^u$-,or $R^{9u}O$-, wherein each $R^{9u}$ is as previously defined;

$R^{10u}$ and $R^{11u}$ are each independently H or alkyl; or $R^{10u}$ is H and $R^{11u}$ is alkyl which is substituted by OH, SH, $SCH_3$, $NH_2$, arylalkyl-OCONH-, $NH_2CO$-, $CO_2H$, guanidino, aryl or heterocyclyl; or the two groups $R^{10u}$ and $R^{11u}$ are joined together to form, with the carbon atom to which they are attached, a 5 or 6 membered carbocyclic ring which may be saturated or mono-unsaturated and which may optionally be substituted by alkyl or fused to a further 5 or 6 membered saturated or unsaturated carbocyclic ring;

or $R^{10u}$ is H, $n^u$ is 0 and $R^{8u}$ and $R^{11u}$ are linked to form a 2-(N-$COR^{9u}$-4-aminopyrrolidinyl) group;

$R^{12u}$ is $R^{13u}R^{14u}NCO$-, $R^{9u}OCH_2$- or heterocyclyl, wherein $R^{9u}$ is as previously defined;

$R^{13u}$ and $R^{14u}$ are each independently H, alkyl, cycloalkyl, aryl, arylalkyl, alkoxyalkyl, aminoalkyl, heterocyclyl or heterocyclylalkyl; or the two groups $R^{13u}$ and $R^{14u}$ are taken together to form, with the nitrogen to which they are attached, a pyrrolidinyl, piperidino, morpholino, piperazinyl, N-alkylpiperazinyl, pyrrolyl, imidazolyl, pyrazolyl or triazolyl group;

$n^u$ is 0 or 1;

$p^u$ is 0 or an integer of from 1 to 6;

and $q^u$ is 0, 1 or 2;

and pharmaceutically acceptable salts thereof and bioprecursors therefor.

The above descriptions of NEP inhibitors suitable for use in the present invention were taken from the noted patents or applications. Reference should be made to such patents and applications for their full disclosures of such classes and specific compounds within those classes, and as to any typographical errors or the like which may have occurred in transcription. Also, in describing such suitable NEP inhibitors, the superscript letters a-i, k and q-u were included to distinguish among the various classes of compounds and the variable substituent groups thereof.

Other suitable NEP inhibitors include SQ 28603 (N-[2-(mercaptomethyl)-1-oxo-3-phenylpropyl]-$\beta$-alanine), disclosed in South African Patent Application 84/0670; UK 69578 (cis-4-[[[1-[2-carboxy-3-(2-methoxyethoxy)propyl]-cyclopentyl]carbonyl]amino]-cyclohexanecarboxylic acid) and its active enantiomer(s); thiorphan and its enantiomers; retro-thiorphan; phosphoramidon; SQ 29072 (7-[[2-(mercaptomethyl)-1-oxo-3-phenylpropyl]amino]-heptanoic acid); and MDL 100,173, having the formula

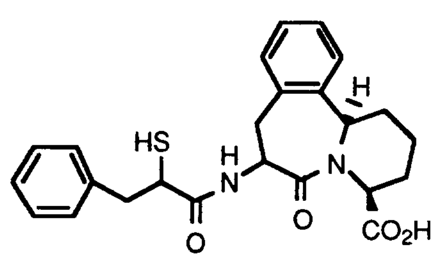

Also suitable for use are any pro-drug forms of the above-listed NEP inhibitors, e.g., compounds in which one or more carboxylic acid groups are esterified.

Another aspect of the invention is the administration of a combination of an ACE inhibitor and an NEP inhibitor. While the use of any ACE inhibitor is contemplated, the following Table I lists ACE inhibitors preferred for use in the combination of this invention:

## TABLE I
## PREFERRED ACE INHIBITORS

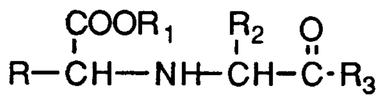

| | R | $R_1$ | $R_2$ | $R_3$ |
|---|---|---|---|---|
| spirapril | $C_6H_5CH_2CH_2$- | Et | $CH_3$ | 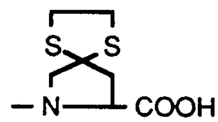 |
| enalapril | $C_6H_5CH_2CH_2$- | Et | $CH_3$ | prolyl |
| ramipril | $C_6H_5CH_2CH_2$- | Et | $CH_3$ | 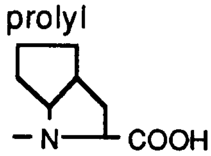 |
| perindopril | $CH_3CH_2CH_2$- | Et | $CH_3$ | 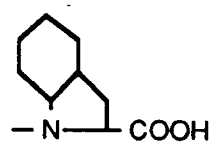 |
| indolapril | $C_6H_5CH_2CH_2$- | Et | $CH_3$ | 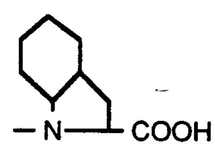 |
| lysinopril | $C_6H_5CH_2CH_2$- | H | $NH_2(CH_2)_4$- | prolyl |
| quinapril | $C_6H_5CH_2CH_2$- | Et | $CH_3$ | 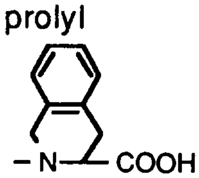 |
| pentopril ($NH = CH_2$) | $CH_3$ | Et | $CH_3$ | 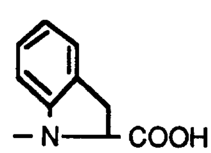 |
| cilazapril | $C_6H_5CH_2CH_2$- | H | 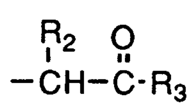 | is 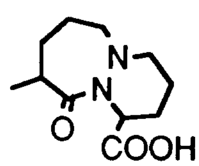 |

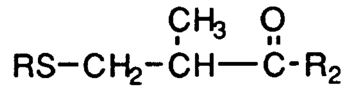

| | R | $R_2$ |
|---|---|---|
| captopril | H | prolyl |
| zofenopril | $C_6H_5CO-$ | 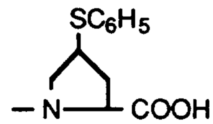 |
| pivalopril | 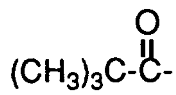 | 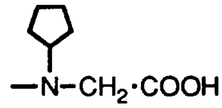 |

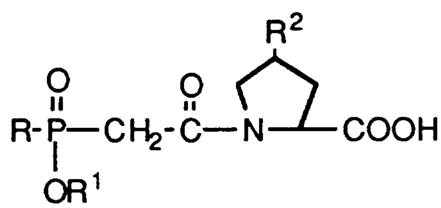

| | R | $R^1$ | $R^2$ |
|---|---|---|---|
| fosinopril | $C_6H_5-(CH_2)_4-$ | 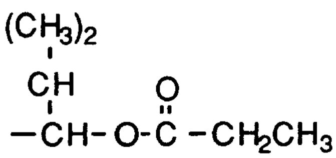 | $C_6H_5-$ |

Still another aspect of the present invention described above relates to the combination of an NEP inhibitor with a natriuretic peptide. As indicated by Needleman et al., N. Eng. J. Med., 314, 13 (1866), p. 828-834, a number of ANP have been isolated so far, all having the same core sequence of 17 amino acids within a cysteine disulfide bridge, but having different N-termini lengths. These peptides represent N-terminal truncated fragments (21-48 amino acids) of a common preprohormone (151 and 152 amino acids for man and rats, respectively). Human, porcine and bovine carboxy-terminal 28-amino acid peptides are identical and differ from similar peptides in rats and mice in that the former contain a methionine group at position 12 while the latter contain isoleucine. Various synthetic analogs of naturally occuring ANF's also have been found to have comparable biological activity. Examples of ANFs contemplated for use in this invention are a human AP 21 (atriopeptin I), a human AP 28, a human AP 23 (atriopeptin II or APII), a human AP 24, a human AP 25, a human AP 26, a human AP 33, and the corresponding rat sequence of each of the above wherein Met 12 is Ile. BNP have been found to be divergent among species, while mammalian CNP is highly conserved among species. See Suga et. al., Endocrinology, 130, 1 (1992) p. 229-239.

The effectiveness of NEP inhibitors in treating atherosclerosis, restenosis induced by angioplasty or vascular reconstructive surgery, glomerulonephritis, glomerulosclerosis or other diseases involving vascular cell proliferation in an animal model can be demonstrated by the following procedure. The left carotid

arteries of male spontaneously hypertensive rats (300-350g) were deendothelialized and injured by the balloon catheter method of Baumgartner, as described in Z. Gesamte Exp. Med., 137 (1963) 227, as modified by Clowes, A.W. et al in Lab. Invest., 49 (1983) 208-215. Briefly, rats were anesthetized with Metofane (methoxyflurane) and a 2 Fr Fogarty embolectomy catheter was inserted into the left external carotid artery. The catheter was advanced 5 cm, inflated and retracted 3 times, turning the catheter 90° each time. We have confirmed by scanning electron microscopy and Evans blue staining that this procedure causes complete deendothelialization of the common carotid artery. Animals were autopsied following exsanguination with ketamine (40 mg/kg) plus xylazine (10 mg/kg) i.m. anesthesia at specified timepoints following balloon injury.

The NEP inhibitor, typically at 10-90 mg/kg bid in 0.4% aqueous methylcellulose vehicle or vehicle alone, was given to groups of SHRs (n = 8-10/group) beginning 6 days prior to balloon injury and carotid artery intimal proliferation was evaluated at 14 days post-injury.

Standard samples of the control right and balloon injured left carotid arteries were taken for DNA and morphometric analyses. The carotid artery DNA content was determined in 5 mm samples taken 5 mm from their aortic origin by the method of La Barca, as reported in Anal. Biochem., 102 (1980) 344. The DNA results were expressed as the ratio of the left or ballooned carotid to the right control carotid. Three samples were taken for histology at 2.5 mm intervals cephalad from the DNA sample from each carotid artery and fixed in 4% paraformaldehyde containing 10% sucrose (wt/vol). Paraffin-embedded sections were stained with the Gomori trichrome-aldehyde fuchsin stain and computer-assisted morphometric analyses were performed using a Bioquant System IV image analyzer (R&M Biometrics Inc.). Morphometric measurements included: maximal intimal thickness (mm), intimal cross sectional area ($mm^2$), and intimal cross sectional area/medial cross sectional area.

The effect of natriuretic peptides can be similarly tested by administering the peptide by continuous intravenous infusion using techniques and apparatus well known in the art. When testing the combinations of this invention, the NEP inhibitor may be administered simultaneously with the ACE inhibitor or the natriuretic peptide, or the combination of drugs can be administered sequentially, with either being administered first, using the appropriate method of administration for each compound.

A variety of pharmaceutical dosage forms are suitable for administration of the drugs. Oral or parenteral administration is preferred, although mechanical delivery systems such as transdermal and intra-catheter dosage forms are also contemplated.

The typical daily dosage of the NEP inhibitor for this invention, when admnistered alone for treatment or prevention of atherosclerosis and restenosis, is about 0.3 mg/kg to about 100 mg/kg of mammalian weight per day administered in single or divided doses; for the natriuretic peptide alone, the typical dosage 0.001 to 0.1 mg natriuretic peptide/kg of mammalian weight per day, in single or divided dosages; for the combination of an NEP inhibitor and a natriuretic peptide, the typical dosage is 0.3 to 100 mg of NEP inhibitor/kg mammalian weight per day in single or divided dosages plus 0.001 to 0.1 mg natriuretic peptide/kg of mammalian weight per day, in single or divided dosages; and for the combination of an NEP inhibitor and an ACE inhibitor, the typical dosage is 0.3 to 100 mg of NEP inhibitor/kg mammalian weight per day in single or divided dosages plus 0.1 to 30 mg ACE inhibitor/kg of mammalian weight per day in single or divided dosages. The exact dose of any natriuretic peptide, NEP inhibitor or combination to be administered is determined by the attending clinician and is dependent on the potency of the compound administered, the age, weight, condition and response of the patient.

Generally, in treating humans suffering from, or preventing the occurence of, atherosclerosis, restenosis induced by angioplasty or vascular reconstructive surgery, glomerulonephritis, glomerulosclerosis or other diseases involving vascular cell proliferation, the NEP inhibitors of this invention can be administered alone in dosage ranges of about 10 to about 500 mg NEP inhibitor per dose given 1 to 4 times a day; natriuretic peptides can be administered alone in dosage ranges of about 0.01 to about 1 mg natriuretic peptide given 1 to 6 times a day (total daily dosage range of .01 to 6 mg/day); for the combination of an NEP inhibitor and a natriuretic peptide, the dosage ranges are about 10 to about 500 mg NEP inhibitor per dose given 1 to 4 times a day and about 0.01 to about 1 mg natriuretic peptide given 1 to 6 times a day (total daily dosage range of 10 to 2000 mg day and .01 to 6 mg/day, respectively); and for the combination of an NEP inhibitor and an ACE inhibitor, the dosage ranges are about 10 to about 500 mg NEP inhibitor per dose given 1 to 4 times a day and about 5 to about 50 mg ACE inhibitor given 1 to 3 times a day (total daily dosage range of 10 to 2000 mg/day and 5 to 150 mg/day, respectively). The drug or combination of drugs is preferably administered in a pharmaceutically acceptable carrier, e.g. for oral or parenteral administration. The combinations of drugs may be co-administered in a single composition, or components of the combination therapy may be administered separately. Where the components are administered separately, any convenient combination of dosage forms may be used, e.g. oral NEP inhibitor/parenteral natriuretic peptide, oral

NEP inhibitor/parenteral ACE inhibitor, parenteral NEP inhibitor/parenteral natriuretic peptide, parenteral NEP inhibitor/parenteral ACE inhibitor. Where the components of a combination are administered separately, the number of doses of each component given per day may not necessarily be the same, e.g. where one component may have a greater duration of activity, it will therefore need to be administered less frequently.

Typical oral formulations for compounds used in this invention include tablets, capsules, syrups, elixirs and suspensions. Typical injectable formulations for compounds used in this invention include solutions and suspensions.

The typical pharmaceutically acceptable carriers for use in the formulations described above are exemplified by: sugars such as lactose, sucrose, and sorbitol; starches such as cornstarch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calcium sulfate; polyvinylpyrrolidone; polyvinyl alcohol; stearic acid; alkaline earth metal stearates such as magnesium stearate and calcium stearate; stearic acid; vegetable oils such as peanut oil, cottonseed oil, sesame oil, olive oil and corn oil; non-ionic, cationic and anionic surfactants; ethylene glycol polymers; betacyclodextrin; fatty alcohols; and hydrolyzed cereal solids, as well as other non-toxic compatible fillers, binders, disintegrants, buffers, preservatives, antioxidants, lubricants, flavoring agents, and the like commonly used in pharmaceutical formulations.

Since the present invention includes treatment with a combination of active ingredients, wherein said active ingredients may be administered separately, the invention also relates to combining separate pharmaceutical compositions in kit form. That is, two kits are contemplated, each combining two separate units: an NEP inhibitor pharmaceutical composition and a natriuretic peptide pharmaceutical composition in one kit and an NEP inhibitor pharmaceutical composition and an ACE inhibitor pharmaceutical composition in a second kit. The kit form is particularly advantageous when the separate components must be administered in different dosage forms (e.g. oral and parenteral) or are administered at different dosage intervals.

Using the methods described above, the results tabulated below were obtained for the following compounds and combinations of compounds:

Experiments 1 and 1A: NEP inhibitor -N-[N-[(L)-[1-[(2,2-dimethyl-1,3-dioxolan-4-yl)methoxy]carbonyl]-2-phenylethyl]-L-phenylalanyl]-ß-alanine (compound A);

Experiments 2 and 2A: NEP inhibitor -N-[2-acetylthiomethyl-3-(2-methylphenyl)-propionyl]-methionine ethyl ester (compound B):

Experiment 3: natriuretic peptide - 28-amino acid rat ANP (Peninsula Laboratories, Inc., Belmont, CA) (compound C);

Experiment 4: the combination of Compounds B and C;

Experiments 5 and 5A: the combination of Compound A and the ACE inhibitor spirapril (compound D) - see Experiments 1 and 1A, respectively, for data on compound A alone and the control.

| Compound | Maximal Intimal Thickness (mm) | Intimal Area ($mm^2$) | Intimal Area/ Medial Area | Carotid DNA (ballooned/ control) |
|---|---|---|---|---|
| | | | | |
| Exp. 1 | | | | |
| 14 day control | 0.132 ±0.007 | 0.113 ±0.008 | 0.815 ±0.058 | 4.98 ±0.60 |
| compound A (90 mpk)* | 0.104 ±0.011 | 0.99 ±0.010 | 0.653 ±0.067 | 3.16 ±0.35 |
| | | | | |
| Exp. 1A | | | | |
| 14 day control | 0.147 ±0.010 | 0.144 ±0.010 | 1.006 ±0.062 | 4.04 ±0.57 |
| compound A (90 mpk)* | 0.110 ±0.010 | 0.099 ±0.010 | 0.692 ±0.066 | 4.60 ±0.67 |
| | | | | |
| Exp. 2 | | | | |
| 14 day control | 0.126 ±0.009 | 0.121 ±0.009 | 0.845 ±0.054 | 3.94 ±0.62 |
| compound B (30 mpk)* | 0.085 ±0.009 | 0.071 ±0.010 | 0.441 ±0.052 | 3.47 ±0.40 |
| | | | | |
| Exp. 2A | | | | |
| 14 day control | 0.140 ±0.007 | 0.126 ±0.007 | 0.962 ±0.045 | 4.14 ±0.42 |
| compound B (10 mpk)* | 0.112 ±0.012 | 0.106 ±0.010 | 0.719 ±0.062 | 3.12 ±0.22 |

| Compound | Maximal Intimal Thickness (mm) | Intimal Area ($mm^2$) | Intimal Area/ Medial Area | Carotid DNA (ballooned/ control) |
|---|---|---|---|---|
| | | | | |
| Exp. 3 | | | | |
| 14 day control | 0.118 ±0.013 | 0.100 ±0.011 | 0.721 ±0.075 | 3.71 ±0.16 |
| compound C (0.3 µg/hr)** | 0.074 ±0.011 | 0.067 ±0.014 | 0.476 ±0.075 | 3.23 ±0.30 |
| | | | | |
| Exp. 4 | | | | |
| 14 day control | 0.114 ±0.008 | 0.106 ±0.008 | 0.799 ±0.053 | 5.57 ±0.89 |
| compound C (0.3 µg/hr)** | 0.071 ±0.012 | 0.060 ±0.011 | 0.437 ±0.077 | 3.44 ±0.73 |
| compound B (10 mpk)* | 0.095 ±0.012 | 0.085 ±0.009 | 0.592 ±0.067 | 4.38 ±0.73 |
| compound C (0.3 µg/hr)** plus compound B (10 mpk)* | 0.083 ±0.011 | 0.069 ±0.011 | 0.471 ±0.071 | 3.25 ±0.42 |
| | | | | |
| Exp. 5 | | | | |
| compound A (90 mpk)* plus compound D (10 mpk)*** | 0.061 ±0.009 | 0.051 ±0.009 | 0.326 ±0.051 | 1.97 ±0.40 |

| Compound | Maximal Intimal Thickness (mm) | Intimal Area ($mm^2$) | Intimal Area/ Medial Area | Carotid DNA (ballooned/ control) |
|---|---|---|---|---|
| | | | | |
| Exp. 5A | | | | |
| compound D (10 mpk)*** | 0.082 ±0.010 | 0.064 ±0.005 | 0.500 ±0.049 | 1.94 ±0.32 |
| compound A (90 mpk)* plus compound D (10 mpk)*** | 0.083 ±0.009 | 0.063 ±0.006 | 0.451 ±0.046 | 2.98 ±0.55 |

[SHR rats (n=8-10/group) with 3 histology and 1 DNA sample per carotid artery (means ± SEM).]

* Dosages are expressed in mg/kg (mpk) b.i.d.

** Dosages are expressed in µg/hr of continuous i.v. infusion

*** Dosages are expressed in mg/kg (mpk) once a day

## Claims

1. A pharmaceutical composition for treating or preventing myointimal proliferation in atherosclerosis, restenosis induced by angioplasty or vascular reconstructive surgery, glomerulonephritis, glomerulosclerosis or other diseases involving vascular cell proliferation, comprising a natriuretic peptide, a neutral endopeptidase inhibitor, a combination of a neutral endopeptidase inhibitor and a natriuretic peptide, or a combination of a neutral endopeptidase inhibitor and an angiotensin converting enzyme inhibitor, in a pharmaceutically acceptable carrier.

2. A pharmaceutical composition of claim 1 wherein the natriuretic peptide, when present, is selected from the group consisting of α human AP 21, α human AP 28, α human AP 23, α human AP 24, α human AP 25, α human AP 26, α human AP 33, the corresponding atrial peptides wherein the methionine at position 12 is replaced by isoleucine, human brain natriuretic peptide and C-type natriuretic peptide.

3. A pharmaceutical composition according to claim 1 or 2 wherein the neutral endopeptidase inhibitor, when present, is selected from the group consisting of:
N-[N-[(L)-[1-[(2,2-dimethyl-1,3-dioxolan-4-yl)methoxy]carbonyl]-2-phenylethyl]-L-phenylalanyl]-β-alanine;
N-[N-[(L)-1-carboxy-2-phenylethyl]-L-phenylalanyl]-β-alanine;
N-[2-acetylthiomethyl-3-(2-methylphenyl)propionyl]methionine ethyl ester;
N-[2-mercaptomethyl-3-(2-methylphenyl)propioyl]methionine;
N-[2(S)-mercaptomethyl-3-(2-methylphenyl)propanoyl]-(S)-isoserine;
N-(S)-[3-mercapto-2-(2-methylphenyl)propionyl]-(S)-2-methoxy-β-alanine;
N-[1-[[1(S)-benzyloxycarbonyl-3-phenylpropyl]amino]cyclopentylcarbonyl]-(S)-isoserine;
N-[1-[[1(S)-carbonyl-3-phenylpropyl]amino]cyclopentylcarbonyl]-(S)-isoserine;
1,1'-[dithiobis-[2(S)-(2-methylbenzyl)-1-oxo-3,1-propanediyl]]-bis-(S)-isoserine;
1,1'-[dithiobis-[2(S)-(2-methylbenzyl)-1-oxo-3,1-propanediyl]]-bis-(S)-methionine;
N-(3-phenyl-2-(mercaptomethyl)propionyl)-(S)-4-(methylmercapto)methionine;

N-[2-acetylthiomethyl-3-phenylpropionyl]-3-aminobenzoic acid;
N-[2-mercaptomethyl-3-phenylpropionyl]-3-aminobenzoic acid;
N-[1-(2-carboxy-4-phenylbutyl)cyclopentanecarbonyl]-(S)-isoserine;
N-[1-(acetylthiomethyl)cyclopentanecarbonyl]-(S)-methionine ethyl ester;
3(S)-[2-(acetylthiomethyl)-3-phenylpropionyl]amino-ϵ-caprolactam;
N-(R,S)-[2-mercaptomethyl-3-(3-fluorophenyl)propionyl]glycine;
N-(R,S)-[2-mercaptomethyl-3-(3-fluorophenyl)propionyl]-(S)-alanine;
N-(R,S)-[2-mercaptomethyl-3-(3,4-difluorophenyl)propionyl]-(S)-alanine;
N-(R,S)-[2-mercaptomethyl-3-(3,5-difluorophenyl)propionyl]-glycine;
N-(R,S)-[2-mercaptomethyl-3-(3,5-difluorophenyl)propionyl]-(S)-alanine;
N-(S)-[1-oxo-2-(acetylthiomethyl)-3-(3,4-methylenedioxyphenyl)-propionyl]-(S)-alanine benzyl ester;
N-(S)-[1-oxo-2-(acetylthiomethyl)-3-(3,4-methylenedioxyphenyl)-propionyl]-(S)-alanine;
SQ 28603; UK 69578; thiorphan; retro-thiorphan; phosphoramidon; SQ 29072; and MDL 100,173.

**4.** A pharmaceutical composition of claim 1 or 3 wherein the angiotensin converting enzyme inhibitor, when present, is selected from: spirapril, enalapril, ramipril, perindopril, indolapril, lysinopril, quinapril, pentopril, cilazapril, captopril, zofenopril, pivalopril and fosinopril.

**5.** A method of treating or preventing myointimal proliferation in atherosclerosis, restenosis, induced by angioplasty or vascular reconstructive surgery, glomerulonephritis, glomerulosclerosis or other diseases involving vascular cell proliferation, comprising administering an effective amount of a natriuretic peptide, a neutral endopeptidase inhibitor, a combination of a neutral endopeptidase inhibitor and a natriuretic peptide, or a combination of a neutral endopeptidase inhibitor and an angiotensin converting enzyme inhibitor, to a mammal in need of such treatment.

**6.** A method of claim 5 wherin the natriuretic peptide, when present, is selected from the group consisting of α human AP 21, α human AP 28, α human AP 23, α human AP 24, α human AP 25, α human AP 26, α human AP 33, the corresponding atrial peptides wherein the methionine at position 12 is replaced by isoleucine, human brain natriuretic peptide and human C-type natriuretic peptide.

**7.** A method of claim 5 or 6 wherein the neutral endopeptidase inhibitor, when present, is selected from the group consisting of:
N-[N-[(L)-[1-[(2,2-dimethyl-1,3-dioxolan-4-yl)methoxy]carbonyl]-2-phenylethyl]-L-phenylalanyl]-ß-alanine;
N-[N-[(L)-1-carboxy-2-phenylethyl]-L-phenylalanyl]-ß-alanine;
N-[2-acetylthiomethyl-3-(2-methylphenyl)propionyl]methionine ethyl ester;
N-[2-mercaptomethyl-3-(2-methylphenyl)propioyl]methionine;
N-[2(S)-mercaptomethyl-3-(2-methylphenyl)propanoyl]-(S)-isoserine;
N-(S)-[3-mercapto-2-(2-methylphenyl)propionyl]-(S)-2-methoxy-ß-alanine;
N-[1-[[1(S)-benzyloxycarbonyl-3-phenylpropyl]amino]cyclopentylcarbonyl]-(S)-isoserine;
N-[1-[[1(S)-carbonyl-3-phenylpropyl]amino]cyclopentylcarbonyl]-(S)-isoserine;
1,1'-[dithiobis-[2(S)-(2-methylbenzyl)-1-oxo-3,1-propanediyl]]-bis-(S)-isoserine;
1,1'-[dithiobis-[2(S)-(2-methylbenzyl)-1-oxo-3,1-propanediyl]]-bis-(S)-methionine;
N-(3-phenyl-2-(mercaptomethyl)-propionyl)-(S)-4-(methyl-mercapto)methionine;
N-[2-acetylthiomethyl-3-phenylpropionyl]-3-aminobenzoic acid;
N-[2-mercaptomethyl-3-phenylpropionyl]-3-aminobenzoic acid;
N-[1-(2-carboxy-4-phenylbutyl)cyclopentanecarbonyl]-(S)-isoserine;
N-[1-(acetylthiomethyl)cyclopentanecarbonyl]-(S)-methionine ethyl ester;
3(S)-[2-(acetylthiomethyl)-3-phenylpropionyl]amino-ϵ-caprolactam;
N-(R,S)-[2-mercaptomethyl-3-(3-fluorophenyl)propionyl]glycine;
N-(R,S)-[2-mercaptomethyl-3-(3-fluorophenyl)propionyl]-(S)-alanine;
N-(R,S)-[2-mercaptomethyl-3-(3,4-difluorophenyl)propionyl]-(S)-alanine;
N-(R,S)-[2-mercaptomethyl-3-(3,5-difluorophenyl)propionyl]-glycine;
N-(R,S)-[2-mercaptomethyl-3-(3,5-difluorophenyl)propionyl]-(S)-alanine;
N-(S)-[1-oxo-2-(acetylthiomethyl)-3-(3,4-methylenedioxyphenyl)-propionyl]-(S)-alanine benzyl ester;
N-(S)-[1-oxo-2-(acetylthiomethyl)-3-(3,4-methylenedioxyphenyl)-propionyl]-(S)-alanine;
SQ 28603; UK 69578; thiorphan; retro-thiorphan; phosphoramidon; SQ 29072; and MDL 100,173.

8. A method of claim 5 or 7 wherein the angiotensin converting enzyme inhibitor, when present, is selected from: spirapril, enalapril, ramipril, perindopril, indolapril, lysinopril, quinapril, pentopril, cilazapril, captopril, zofenopril, pivalopril and fosinopril.

9. A method of treating or preventing myointimal proliferation in atherosclerosis, restenosis induced by angioplasty or vascular reconstructive surgery, glomerulonephritis, glomerulosclerosis or other diseases involving vascular cell proliferation, comprising administering a composition according to claim 1 to a mammal in need of such treatment.

10. A kit comprising in separate containers in a single package pharmaceutical compositions for use in combination to treat or prevent myointimal proliferation in atherosclerosis, restenosis induced by angioplasty or vascular reconstructive surgery, glomerulonephritis, glomerulosclerosis or other diseases involving vascular cell proliferation in mammals which comprises in one container a pharmaceutical composition comprising a neutral endopeptidase inhibitor and in a second container a pharmaceutical composition comprising a natriuretic peptide or an angiotensin converting enzyme inhibitor.

11. The use of a natriuretic peptide, a neutral endopeptidase inhibitor, a combination of a neutral endopeptidase inhibitor and a natriuretic peptide, or a combination of a neutral endopeptidase inhibitor and an angiotensin converting enzyme inhibitor for the manufacture of a medicament for treating or preventing myointimal proliferation in atherosclerosis, restenosis induced by angioplasty or vascular reconstructive surgery, glomerulonephritis, glomerulosclerosis or other diseases involving vascular cell proliferation.

12. A process for preparing a pharmaceutical composition as claimed in claim 1 which comprises admixing a compound or combinaton of claim 1 with a pharmaceutical carrier.

European Patent Office

# EUROPEAN SEARCH REPORT

Application Number

EP 92 11 5681
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 254 032 (SCHERING CORPORATION)<br>* page 1 - page 3; tables 3,4 * | 1-4,10, 12 | A61K37/64<br>A61K37/02<br>A61K37/24 |
| D,X | WO-A-9 107 386 (SCHERING CORPORATION)<br>* claims 1-12,14 * | 1-4,10, 12<br>1 | |
| D,P, Y | EP-A-0 459 318 (E.R. SQUIBB & SONS, INC.)<br>* claims 1-8 * | 5-9,11 | |
| X | J. CLIN. INVEST.<br>vol. 86, no. 5, November 1990,<br>pages 1690 - 1697;<br>H. ITOH ET AL.: 'Atrial natriuretic polypeptide inhibits hypertrophy of vascular smooth muscle cells'<br>*as a whole, in particular page 1690 and 1695* | 5-9,11 | |
| X | CLINICAL RESEARCH<br>vol. 39, no. 2, 1991,<br>page 286A;<br>H. ITOH ET AL.: 'A novel action of atrial natriuretic polypeptide (ANP) as an antigrowth factor of endothelial cells'<br>& Joint Meeting of the Assoc. of Am. Physicians, the Am. Soc. for Clin. Investigation, and the Am. Fed. for Clin. Research<br>Seattle, Washington, USA, May 3-6, 1991<br>* abstract * | 5-9,11 | **TECHNICAL FIELDS SEARCHED (Int. Cl.5)**<br>A61K |
| X | CLINICAL RESEARCH<br>vol. 38, no. 1, 1990,<br>page 162A;<br>C. WESTENFELDER ET AL.: 'Atrial natriuretic factor (ANF) is a potent antimitogen in glomerular mesangial (MSC) and aortic smooth muscle (SMC) cells'<br>* abstract * | 5-9,11 | |

The present search report has been drawn up for all claims

| Place of search | Date of completion of the search | Examiner |
|---|---|---|
| MUNICH | 21 DECEMBER 1992 | FOERSTER W.K. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 92 11 5681
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | CLINICAL RESEARCH vol. 36, no. 3, 1988, page 259A; S.P. BAGBY ET AL: 'Angiotensin II (AII) stimulates proliferation of aortic endothelial cells' * abstract * | 5-9,11 | |
| Y | CLINICAL RESEARCH vol. 37, no. 2, 1989, page 286A; G.N. POWELL ET AL.: 'Smooth muscle cell proliferation in response to vascular injury is suppressed by cilazapril, an angiotensin converting enzyme inhibitor' * abstract * | 5-9,11 | |
| P,X | HYPERTENSION vol. 19, no. 6/2, June 1992, pages 758 - 761; H.ITOH ET AL.: 'atrial natriuretic polypeptide as a novel antigrowth factor of endothelial cells' *as a whole, in particular see page 758 and 760* | 5-9,11 | TECHNICAL FIELDS SEARCHED (Int. Cl.5) |
| P,X | AM. J. PHYSIOL. vol. 262, no. 6/2, June 1992, pages 911 - 918; R. G. APPEL: 'Growth-regulatory properties of atrial natriuretic factor' * the whole document * | 5-9,11 | |

The present search report has been drawn up for all claims

| Place of search | Date of completion of the search | Examiner |
|---|---|---|
| MUNICH | 21 DECEMBER 1992 | FOERSTER W.K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document